# EUROPEAN PATENT SPECIFICATION

(11) **EP 3 128 908 B1**
(45) Date of publication and mention of the grant of the patent: **22.05.2019**
(21) Application number: 15776710.4
(22) Date of filing: 10.04.2015
(51) Int. Cl.: A61B 5/055, A61B 8/12, G01N 29/00

(54) **INTERVENTIONAL MRI COMPATIBLE MEDICAL DEVICE, SYSTEM, AND METHOD**
INTERVENTIONELLE MRT-KOMPATIBLE MEDIZINISCHE VORRICHTUNG, SYSTEM UND VERFAHREN
DISPOSITIF MÉDICAL COMPATIBLE AVEC UNE IRM INTERVENTIONNELLE, SYSTÈME ET PROCÉDÉ

(30) Priority: 10.04.2014 US 201461977700 P
(43) Date of publication of application: 15.02.2017
(73) Proprietor: Georgia Tech Research Corporation, Atlanta, GA 30332-0415 (US); Bogazici University, 34342 Bebek - Istanbul (TR)
(72) Inventor: DEGERTEKIN, Fahrettin Levent, Atlanta, GA 30332-0415 (US); KOCATURK, Ozgur, 34342 Bebek Istanbul (TR)
(74) Representative: Dekker-Garms, Alwine Emilie
(86) International application number: PCT/US2015/025414
(87) International publication number: WO 2015/157695

(56) References cited:
- WO-A1-02/086526
- WO-A1-2005/103747
- WO-A1-2010/039950
- US-A- 5 135 295
- US-A- 5 882 305
- US-A1- 2005 203 378
- US-A1- 2010 022 867
- US-A1- 2013 090 563
- US-B2- 6 470 205

## Description

### CROSS-REFERENCE TO RELATED APPLICATIONS

This application claims the benefit of US Provisional Application No. 61/977,700 filed 10 April 201.

### BACKGROUND OF THE INVENTION

### 1. Field of the Invention

The present invention relates generally to MRI compatible devices, systems and methods, and more specifically to a clinical-grade active catheter device that does not need long conductor transmission lines for active device visualization under MRI.

### 2. Description of Related Art

Interventional cardiovascular magnetic resonance imaging (MRI) has the potential as an ionizing radiation-free alternative to conventional X-ray guided catheterization, but its use has so far been limited in clinical settings because of lack of safe and conspicuous catheter devices. Engineering safe and conspicuous MRI catheters is especially challenging because of the combination of electrical and mechanical requirements and profile constraints discussed above.

An important problem for interventional MRI device safety is the radio frequency (RF) induced heating over incorporated long conductive materials. The resonant length of the conductor is a main contributor to heating, but it is a complex problem since partial guide wire insertion from air into the body creates complex resonance patterns during clinical use as the operator moves the device. While detuning circuitry can mitigate inductive coupling between the guide wire and RF transmitter, capacitive coupling is more difficult to suppress.

A second problem is visibility. Commercial catheters and guide wires used in X-ray based procedures contain metal cores and radiopaque coils at the distal tip to increase fluoroscopic visibility. The interventionist needs to visualize the entire shaft and exact tip location in order to navigate vascular structures safely. Passively-visualized devices have poor contrast dependent on the device orientation relative to the main magnetic field and often create image artifacts. Actively-visualized devices incorporate receiver coils. Several methods have been developed to limit or minimize the active device heating, including detuning the devices during RF transmission (two channel), the use of RF chokes or transformers.

However none of these techniques can offer active device design that can have clinically acceptable mechanical performance.

As noted, MRI has achieved prominence as a diagnostic imaging modality, and increasingly as an interventional imaging modality. The primary benefits of MRI over other imaging modalities, such as X-ray, include superior soft tissue imaging and avoiding patient exposure to ionizing radiation produced by X-rays. MRI's superior soft tissue imaging capabilities have offered great clinical benefit with respect to diagnostic imaging. Similarly, interventional procedures, which have traditionally used X-ray imaging for guidance, stand to benefit greatly from MRI's soft tissue imaging capabilities. In addition, the significant patient exposure to ionizing radiation associated with traditional X-ray guided interventional procedures is eliminated with MRI guidance.

MRI uses three fields to image patient anatomy: a large static magnetic field, a time-varying magnetic gradient field, and a radio frequency electromagnetic field. The static magnetic field and time-varying magnetic gradient field work in concert to establish both proton alignment with the static magnetic field and also spatially dependent proton spin frequencies (resonant frequencies) within the patient. The RF field, applied at the resonance frequencies, disturbs the initial alignment, such that when the protons relax back to their initial alignment, the RF emitted from the relaxation event may be detected and processed to create an image.

Each of the three fields associated with MRI present safety risks to patients when a medical device is in close proximity to or in contact either externally or internally with patient tissue. One important safety risk is the heating that can result from an interaction between the RF field of the MRI scanner and the medical device (RF-induced heating), especially medical devices which have elongated conductive structures with tissue contacting electrodes, such as electrode wires in pacemaker and implantable cardioverter defibrillator (ICD) leads, guidewires, and catheters. Thus, as more patients are fitted with implantable medical devices, and as use of MRI diagnostic imaging continues to be prevalent and grow, the need for safe devices in the MRI environment increases.

A variety of MRI techniques are being developed as an alternative to X-ray imaging for guiding interventional procedures. For example, as a medical device is advanced through the patient's body during an interventional procedure, its progress may be tracked so that the device can be delivered properly to a target site. Once delivered to the target site, the device and patient tissue can be monitored to improve therapy delivery. Thus, tracking the position of medical devices is useful in interventional procedures. Exemplary interventional procedures include, for example, cardiac electrophysiology procedures including diagnostic procedures for diagnosing arrhythmias and ablation procedures such as atrial fibrillation ablation, ventricular tachycardia ablation, atrial flutter ablation, Wolfe Parkinson White Syndrome ablation, AV node ablation, SVT ablations and the like. Tracking the position of medical devices using MRI is also useful in oncological procedures such as breast, liver and prostate tumor ablations; and urological procedures such as uterine fibroid and enlarged prostate ablations.

In many of the foregoing cases, elongated or large surface area metallic structures may be present in interventional devices that are used during a procedure to deliver therapy or provide a diagnosis, implanted devices that are placed within the body to provide therapy or deliver a diagnosis, or the tools used to deploy or deliver the interventional or implanted device to the patient. Examples of interventional devices having metallic structures may include plaque excision devices, embolic traps, electrophysiology catheters, biopsy needles/tools, and stem cell delivery catheters. Examples of implanted devices having metallic structures may include cochlear implants, pacemakers, implantable cardioverter defibrillators, Insulin pumps, nerve stimulators, lead wires, prosthetic heart valves, hemostatic clips, and non-ferromagnetic stapedial implants. Finally, examples of deployment or delivery tools having metallic structures may include catheters, sheaths, introducers, guidewires, transseptal devices, and trochars.

As appreciated by those skilled in the art, these metallic structures may undergo heating during an MRI scanning process. This heating may be caused by numerous factors, including but not limited to eddy currents from MRI gradient switching, RF induced heating due to electromagnetic interactions between the metallic structure and the MRI transmit coil, and large current densities at metal/tissue interfaces (where heating may occur in both the metallic structure as well as the connected tissue).

WO 2005/103747 A1 discloses a receive coil for magnetic resonance imaging. An optical resonant cavity includes an electro-optically active medium. A radio frequency antenna is coupled with the electro-optically active medium such that reception of a magnetic resonance signal by the radio frequency antenna modulates an optical characteristic of the optical resonant cavity. At least one optical fiber is optically coupled with the optical resonant cavity.

US 2005/0203378 A1 discloses a receive coil for magnetic resonance imaging. The receive coil comprises an electronic assembly which contains an electrical to optical converter which converts the electrical signals received through pickup coil(s) into optical signals. The electrical to optical converter comprises a photo diode.

WO 02/086526 A1 relates to an arrangement for the optical transmission of an MR signal from an MR receiving coil to a detection unit. The light of a light source is applied, by way of an optical fiber, to an electro-optical modulator which consists of an electro-optical material which is arranged between two crossed polarizers. Thus, a light signal which is amplitude modulated around zero is generated, the intensity of said light signal being proportional to the voltage induced in the MR receiving coil. The modulated light signal is then conducted from the electro-optical modulator to the detection unit by way of an optical fiber.

US 2010/0022867 A1 discloses a receiving coil unit for magnetic resonance imaging. The receiving coil unit comprises an optical modulator utilizing an electro-optical effect. The optical modulator includes a flat substrate in the shape of rectangular parallelepiped, the substrate being formed by LiNbO3 crystal and exhibiting an electro-optical effect (Pockels effect).

It is thus an intention of the present invention to provide for a solution to the two main disadvantages noted previously with an interventional MRI device, effectively avoiding radio frequency induced heating issues while providing active device visualization.

### BRIEF SUMMARY OF THE INVENTION

Briefly described, in a preferred form, the present invention comprises an interventional MRI compatible medical device, system, and method comprising an electro-mechanical conversion assembly, a mechanical-optical conversion assembly, and a signal transmitter/detector assembly. With the careful complimentary combination of each of the three assemblies, in an exemplary embodiment, the present invention provides novel and non-obvious MRI safe active receivers without conducting transmission lines and without compromising mechanical performance. The present invention also offers the possibility of combined temperature measurement and optical detuning capability. Conspicuous MRI catheters with these integrated capabilities are a significant advance in the field of interventional MRI by ensuring safe clinical operation.

In an exemplary embodiment, the electro-mechanical conversion assembly comprises a subsystem that converts electrical inputs to elastic waves. This can be accomplished with a receiver and transducer. In an exemplary embodiment, the receiver is a distal loop coil, and the transducer is an ultrasonic transducer, wherein the distal loop coil is electrically connected to the ultrasonic transducer having a comparable profile.

In an exemplary embodiment, the mechanical-optical conversion assembly comprises a subsystem that converts mechanical inputs to optical waves via acousto-optical modulation with an acousto-optical detector. This can be accomplished with an interferometric detection assembly. In an exemplary embodiment, the ultrasonic transducer of the electro-mechanical conversion assembly converts the electrical input from the distal loop coil to elastic waves at one end of an optical fiber with a sensor region, preferably an embedded interferometric detection structure, for example, a fiber Bragg grating (FBG). The elastic waves over the grating result in acousto-optical modulation of the grating.

In an exemplary embodiment, the signal transmitter/detector assembly comprises a laser coupled to the proximal end of the optical fiber that runs along an active catheter shaft, an optical coupler, a photodetector and a spectrum analyzer.

Therefore, the present invention enables an active receiver coil signal to be detected using an RF heating free, dielectric transmission line resulting in a safe and visible catheter. The acousto-optic detection method is very robust and is widely used for measuring ultrasound fields for therapeutic and medical imaging applications and nondestructive testing through ultrasound induced strain measurements. Optical fibers with different FBG configurations are commercially available.

Integration of an acousto-optical detector to an MRI catheter and its successful demonstration as an active receiver with a non-conducting transmission line is a significant step in achieving the goal of fundamentally more effective, more efficient, safer, novel, and radiation-free interventional procedures. Furthermore, it will pave the way to sensing schemes where local temperature and MRI signals can be detected and transmitted over the same optical fiber leading to safer and cost-effective devices.

Other exemplary embodiments of the present invention include a device comprising an optical fiber including a distal end, and a sensor region disposed at the distal end, and an electro-mechanical conversion assembly in communication with the optical fiber, the electro-mechanical conversion assembly including a receiver comprising a coil, and an ultrasonic transducer disposed adjacent to the sensor region, wherein the ultrasonic transducer is in electrical communication with the receiver, and wherein the ultrasonic transducer is configured to modulate (preferably elastically modulate) the sensor region based on electrical input received from the coil.

The optical fiber can comprise at least one proximal end configured for coupling with an external light source for interrogation of the sensor region. The optical fiber cam comprise at least one proximal end configured for coupling with a photodetector to receive interrogation light reflected from the sensor region.

At least the optical fiber and the electro-mechanical conversion assembly can be configured to reduce radio frequency-induced heating of the device when utilized with Magnetic Resonance Imaging (MRI).

The ultrasonic transducer can comprise at least two electrodes and a thin-film piezoelectric material deposited on the optical fiber. The piezoelectric material can comprise zinc oxide (ZnO).

The sensor region can comprise grating. The sensor region can comprise a Fiber Bragg Grating (FBG). The sensor region can comprise two or more Fiber Bragg Grating mirrors.

The coil can comprise one or more loops. The coil can be disposed at the distal end of the optical fiber.

The ultrasonic transducer can be configured to acousto-optically modulate the sensor region. The ultrasonic transducer can be configured to convert electrical input to elastic wave output. A profile associated with the ultrasonic transducer can match a profile associated with the optical fiber.

Another exemplary embodiment of the present invention includes a system comprising an optical fiber including a distal end, a sensor region disposed at the distal end, and at least one proximal end, an electro-mechanical conversion assembly in communication with the optical fiber, the electro-mechanical conversion assembly including a receiver comprising a coil, and an ultrasonic transducer disposed adjacent to the sensor region, wherein the ultrasonic transducer is in electrical communication with the receiver, and wherein the ultrasonic transducer is configured to elastically modulate the sensor region based on electrical input received from the coil, and a mechanical-optical conversion assembly in communication with the at least one proximal end of the optical fiber, the mechanical-optical conversion assembly including a light source coupled to the at least one proximal end of the optical fiber and configured to interrogate the sensor region, and a photodetector coupled to the at least one proximal end of the optical fiber, the photodetector configured to receive interrogation light reflected from the sensor region.

The system can comprise an interventional probe configured for use with Magnetic Resonance Imaging (MRI), and wherein at least the optical fiber and the electro-mechanical conversion assembly are configured to reduce radio frequency-induced heating of the interventional probe.

Another exemplary embodiment of the present invention includes a method comprising interrogating, with a light source, an interventional probe, the interventional probe comprising an optical fiber having a distal end and a sensor region disposed at the distal end, and an electro-mechanical conversion assembly in communication with the optical fiber, the electro-mechanical conversion assembly including: a receiver comprising a coil, and an ultrasonic transducer disposed adjacent to the sensor region, wherein the ultrasonic transducer is in electrical communication with the receiver, and wherein the ultrasonic transducer is configured to elastically modulate the sensor region based on electrical input received from the coil, and detecting, with a photodetector, interrogation light reflected from the sensor region, and outputting a signal corresponding to the detected interrogation light reflected from the sensor region.

The method can further comprise computing a temperature associated with the sensor region based at least in part on a the detected interrogation light reflected from the sensor region.

The method can further comprise selectively adjusting a resonance associated with the coil.

Selectively adjusting the resonance can comprise optically switching a photoresistor or photodiode in communication with the coil.

These and other objects, features and advantages of the present invention will become more apparent upon reading the following specification in conjunction with the accompanying drawing figures.

### BRIEF DESCRIPTION OF THE DRAWINGS

**Fig. 1** is a schematic of an active receiver with coupled acousto-optic modulator transducer and optical fiber transmission line with FBG, according to an exemplary embodiment of the present invention.
**Fig. 2** is a schematic of partial ZnO deposited fiber showing focused waves on the core.

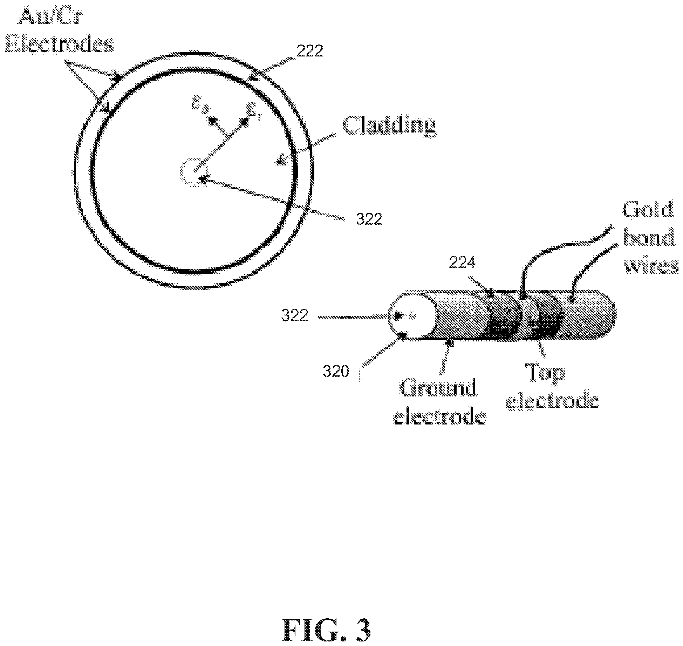

**Fig. 3** is a schematic of an annular ZnO transducer on fiber with bond wire connections.
**Fig. 4** illustrates a 0.08" diameter loop coil implemented for initial designs of the present invention.
**Fig. 5** illustrates a system on which a KLM model based acoustic simulation was performed using ZnO film on a silica optical fiber.
**Fig. 6** is a graph of a calculated insertion loss for the coupled electrical-acoustic system in **Fig. 5****.**
**Fig. 7** is a schematic of the test and measurement setup for an acousto-optic MRI transmission line, according to an exemplary embodiment of the present invention.
**Fig. 8** is a flow-diagram of an method according to an exemplary embodiment of the present invention.

### DETAIL DESCRIPTION OF THE INVENTION

To facilitate an understanding of the principles and features of the various embodiments of the invention, various illustrative embodiments are explained below. Although exemplary embodiments of the invention are explained in detail, it is to be understood that other embodiments are contemplated. Accordingly, it is not intended that the invention is limited in its scope to the details of construction and arrangement of components set forth in the following description or illustrated in the drawings. The invention is capable of other embodiments and of being practiced or carried out in various ways. Also, in describing the exemplary embodiments, specific terminology will be resorted to for the sake of clarity.

It must also be noted that, as used in the specification and the appended claims, the singular forms "a," "an" and "the" include plural references unless the context clearly dictates otherwise. For example, reference to a component is intended also to include composition of a plurality of components. References to a composition containing "a" constituent is intended to include other constituents in addition to the one named.

Also, in describing the exemplary embodiments, terminology will be resorted to for the sake of clarity. It is intended that each term contemplates its broadest meaning as understood by those skilled in the art and includes all technical equivalents which operate in a similar manner to accomplish a similar purpose.

Ranges may be expressed herein as from "about" or "approximately" or "substantially" one particular value and/or to "about" or "approximately" or "substantially" another particular value. When such a range is expressed, other exemplary embodiments include from the one particular value and/or to the other particular value.

Similarly, as used herein, "substantially free" of something, or "substantially pure", and like characterizations, can include both being "at least substantially free" of something, or "at least substantially pure", and being "completely free" of something, or "completely pure".

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

It is also to be understood that the mention of one or more method steps does not preclude the presence of additional method steps or intervening method steps between those steps expressly identified. Similarly, it is also to be understood that the mention of one or more components in a composition does not preclude the presence of additional components than those expressly identified.

The materials described as making up the various elements of the invention are intended to be illustrative and not restrictive. Many suitable materials that would perform the same or a similar function as the materials described herein are intended to be embraced within the scope of the invention. Such other materials not described herein can include, but are not limited to, for example, materials that are developed after the time of the development of the invention.

In an exemplary embodiment, the present invention is a multi-lumen catheter incorporating a micro-lumen for the active receiver and transmission line. The distal coil is connected electrically in parallel with a piezoelectric transducer and excites the transducer with the current induced on the coil. The piezoelectric transducer is directly in contact with the optical fiber over an FBG region and generates acousto-optical modulation signals directly on the fiber. Using a thin film piezoelectric layer, such as a ZnO layer directly deposited on the fiber partially or fully over the circumference, the elastic waves are cylindrically focused on the core of the optical fiber where it is most effective. This technique presents efficient acousto-optic modulation at a target frequency, for example, 66.7MHz, when an acoustic resonance frequency of the optical fiber is used. Enhancements obtain a good SNR from the only few µA current flowing at the distal coil.

In the present invention, techniques described hereinafter are combined and refined in inventive manners. The convenience of electrical-to-mechanical energy conversion with a piezoelectric transducer for receiver coil signal extraction, acousto-optical modulation on the fiber for mechanical-to-optical signal conversion, in some cases increasing the sensitivity through a Bragg grating, and robust optical fibers for signal transmission and detection where the sensitivity can be adjusted by the input laser power. When implemented as the present invention, this combination yields MRI safe active receivers without conducting transmission lines and without compromising mechanical performance. It also offers the possibility of combined temperature measurement and optical detuning capability. Conspicuous MRI catheters with these integrated capabilities are a significant advance in the field of interventional MRI by ensuring safe clinical operation.

Catheter based invasive approaches have been preferred against surgical procedures recently due to the reduction of operation time, patient discomfort, hospitalization time, and procedure related risks. X-ray fluoroscopy is widely used for imaging guidance for the minimally invasive procedures such as treatment of obstructive coronary artery disease, transcatheter aortic valve implantation, peripheral artery atherosclerosis and aneurysm, and structural or congenital heart disease. However, it depicts soft tissue poorly and requires assumptions on device position and orientation based on anatomic landmarks that may be inaccurate. The therapeutic use of MRI in cardiovascular procedures provides superior soft tissue contrast while eliminating the ionization radiation exposure on both patient and operator.

MRI also provides multi slice imaging and allows physiological measurements such as blood flow, temperature, perfusion and motion. The feasibility of endovascular interventional procedures such as renal artery stenosis treatment, abdominal aortic aneurysms treatment, recanalization of carotid chronic total occlusion, atrial septal puncture and transcatheter aortic valve implantation and electrophysiology mapping for atrial fibrillation treatment have been successfully tested on animal models under magnetic resonance imaging.

Due to the intrinsic principle differences the same mechanisms that provide adequate device profile under X-ray imaging make most traditional catheters and guidewires either invisible or not suitable for use under MRI. Interventional cardiovascular devices typically classified by the visualization mechanism called as passive or active. Passive devices rely on material intrinsic properties. The ferromagnetic, paramagnetic or novel contrast agent materials such as 19F are used to improve device visibility. Passive devices are not preferred because they usually cause obstruction of the surrounding anatomy. Active devices incorporate small coils or antennae connected to the scanner on separate channels through long transmission lines for device tracking or profiling purpose.

Clinical grade active endovascular catheters and guidewires are nearing clinical reality, but before moving on clinical trials, the radio frequency induced heating risk over long conductor components of the devices needs to be addressed. The pulsed electromagnetic RF field may induce currents over long conductors in intravascular devices and cause heating especially near the device tips due to resistive losses. Several methods have been developed to limit or minimize the active device heating, including detuning the devices during radio frequency transmission (two channel), the use of RF chokes or transformers in transmission lines to modify the electrical length under MRI. However, although their promising improvements in terms of RF induced heating problem, none of these techniques can offer an active device design that can have clinically acceptable mechanical performance.

Instead of detection of MRI signals directly using conducting electrical transmission lines, one can use dielectric transmission lines if the electrical signal is first transformed in to mechanical or optical signals. Although electrical to mechanical signal conversion can be straightforward using a piezoelectric transducer, the acoustic waves at these RF frequencies (∼64MHz for 1.5T system) tend to converge into a surface wave which would be attenuated significantly when the waveguide surface comes into contact with other materials. As widely used in other MRI safe sensors such as fiber optic microphones, optical fiber is an attractive transmission medium for MRI catheters. Consequently, optical detuning of resonant markers where an optically switched photoresistor or photodiode is used to short circuit the parallel resonance of the coil at the catheter has been demonstrated.

A significant advantage of optical fibers is that they are insensitive to mechanical boundary conditions at their surface since the optical energy is concentrated at the fiber core and they are mechanically very flexible.

Acousto-optical modulation is a technique where mechanical strain generated by an acoustic wave is used to modulate the optical propagation medium through density variations. In its simplest form, an optical fiber can be used as a very broadband hydrophone for measuring ultrasound fields up to 100MHz. More sophisticated applications use the Bragg diffraction phenomenon in different forms.

In addition to bulk wave modulators such as Bragg cells, optical fibers with embedded Bragg gratings are very commonly used in optical sensing of ultrasound fields in kHz to 60MHz range for medical applications as well as nondestructive testing. Strain sensitivity of 40f-strain/√Hz on an aluminum plate and pressure sensitivity down to 1Pa has been demonstrated by subjecting a fiber Bragg grating (FBG) in water using a 3mW laser source at 1549nm and more recently small photo-acoustic sources are detected with over 30MHz bandwidth using pi-phase shifted FPG sensors.

The FBG sensors have the additional capability of detecting ultrasound and local temperature at the same time, which can be very advantageous for safe MRI operation of a catheter. This is simply achieved by monitoring both the slow peak amplitude wavelength variations due to temperature and the fast (1-100MHz) ultrasound induced changes in the received optical intensity. A temperature resolution of 0.2°C is achieved as well as sensitive ultrasound detection at 1.91MHz.

In an exemplary embodiment, the present invention is an active catheter design incorporating a distal loop coil that is electrically connected to an ultrasonic transducer having a comparable profile. The ultrasonic transducer induces ultrasonic waves at the Larmor frequency at the distal end of a dielectric optical fiber that runs along the active catheter shaft. The optical fiber serves as the transmission line instead of a convention conductor, eliminating the RF induced heating.

The dynamic strain generated by the ultrasonic transducer can be measured using optical interferometry by coupling a laser at the proximal end of the optical fiber using the acousto-optical effect. A fiber embedded Bragg reflector grating, for example, can be used for this purpose. The device can also be used for simultaneous temperature measurements among other parameters.

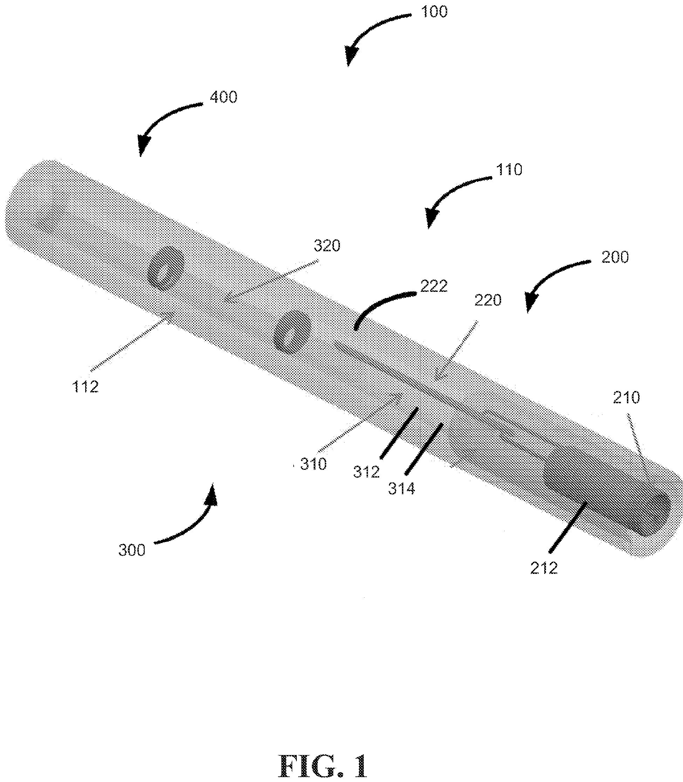

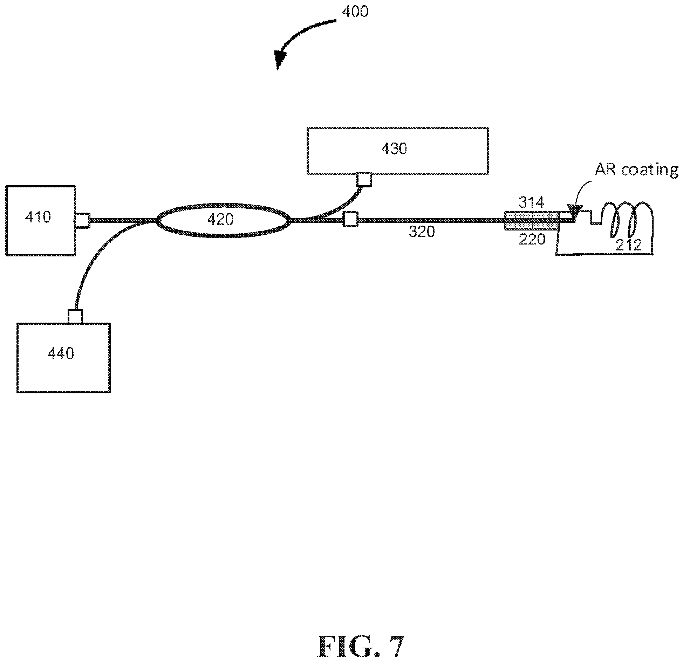

**Fig. 1** illustrates an exemplary embodiment of the present invention being an interventional MRI compatible medical device **100** comprising an electro-mechanical conversion assembly **200** and a mechanical-optical conversion assembly **300** (the signal transmitter/detector assembly **400** is shown in **Fig. 7**). The invention shown in **Fig. 1** takes advantage of advances in high sensitivity phase-shifted FBG based acousto-optical ultrasound detection, direct deposition of piezoelectric thin films on optical fibers for efficient acousto-optical modulation, and MRI compatible catheter design and implementation to realize an active receiver with a safe transmission line.

The present invention shown in **Fig. 1** is a multi-lumen catheter **110** incorporating a micro-lumen **112** for the active receiver and transmission line.

The electro-mechanical conversion assembly **200** comprises a subsystem that converts electrical inputs to elastic waves. This can be accomplished with a receiver **210** and transducer **220.** The receiver **210** can be a distal loop coil **212,** and the transducer **220** is an ultrasonic transducer **222,** wherein the distal loop coil **212** is electrically connected to the ultrasonic transducer **222** having a comparable profile.

The mechanical-optical conversion assembly **300** comprises a subsystem that converts mechanical inputs to optical waves via acousto-optical modulation. This can be accomplished with an interferometric detection assembly **310.** In an exemplary embodiment, the ultrasonic transducer **222** of the electro-mechanical conversion assembly **200** converts the electrical input from the distal loop coil **212** to elastic waves at one end of an optical fiber **320** with a sensor region **312,** preferably an embedded interferometric detection structure, for example, a fiber Bragg grating (FBG) **314.** The elastic waves over the grating result in acousto-optical modulation of the grating **314.**

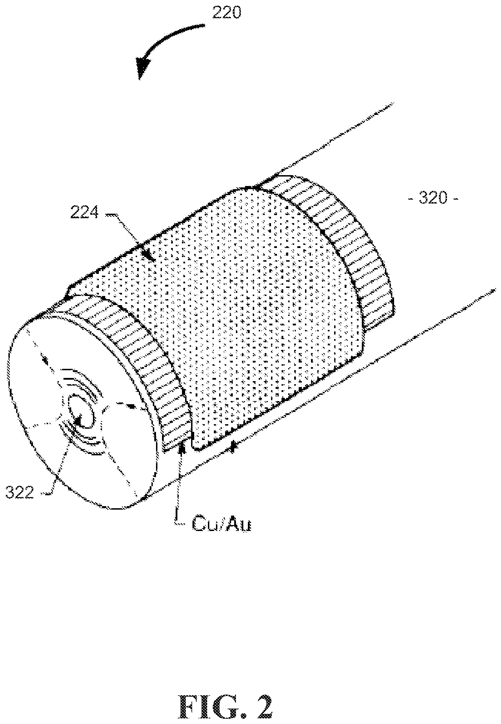

The distal coil **212** is connected electrically in parallel with the piezoelectric transducer **222** and excites the transducer with the current induced on the coil. The piezoelectric transducer is directly in contact with the optical fiber **320** over the FBG region **314** and generates acousto-optical modulation signals directly on the fiber. Using a thin film piezoelectric layer **224,** such as a ZnO layer directly deposited on the fiber partially or fully over the circumference (as shown in **Fig. 2**), the elastic waves will be cylindrically focused on the core **322** of the optical fiber where it is most effective.

This ZnO-based, all-fiber technique has been demonstrated for acousto-optical modulation on a bare fiber by different groups generating up to 100µ-strains, which is ∼10 orders of magnitude larger than the strain level detectable on FBG structures, indicating a very large dynamic range. Most relevant, this technique is used for very efficient acousto-optic modulation at 66.7MHz, very similar to a tested target frequency, when an acoustic resonance frequency of the optical fiber is used.

The present invention utilizes innovative enhancements to obtain a good SNR from the only the few µA current flowing at the distal coil. The present invention providing an active receiver with safe signal transmission lines embodies one or more of the following technical innovations:
- Use of on-fiber deposited thin film piezoelectics for direct acousto-optical modulation. Expensive and laborious bonding and shaping of bulk piezoelectric transducers is avoided.
- Self-focusing transducer geometry and exploitation of acoustic fiber resonances for increased efficiency at Larmor frequency. This frequency can be adjusted for magnet strength by changing fiber diameter using various film deposition and etching techniques and optimizing transducer and electrode thickness for one of the many resonances.
- Use of FBG sensors for high sensitivity strain sensing at ultrasound frequencies. Phase shifted high sensitivity FBGs are especially suitable. Fiber coupled tunable diode lasers in the 1520-1570nm range up to 100mW power are also available.
- Use of optical fiber as a safe and mechanically flexible transmission line for active receiver signal with the possibility of simultaneous active detuning and temperature measurement.
- The present method can also be implemented using bulk piezo attached to a regular optical fiber.
- The present method can also be implemented by placing the acousto optical modulator on a regular fiber region between two FBG mirrors.
- Multiple sensors can be placed over the fiber to learn the position of the catheter in three dimensions along its length in addition to tip location.

### Coupled Active Coil - Ultrasound Transducer Modeling And Implementation On FBG

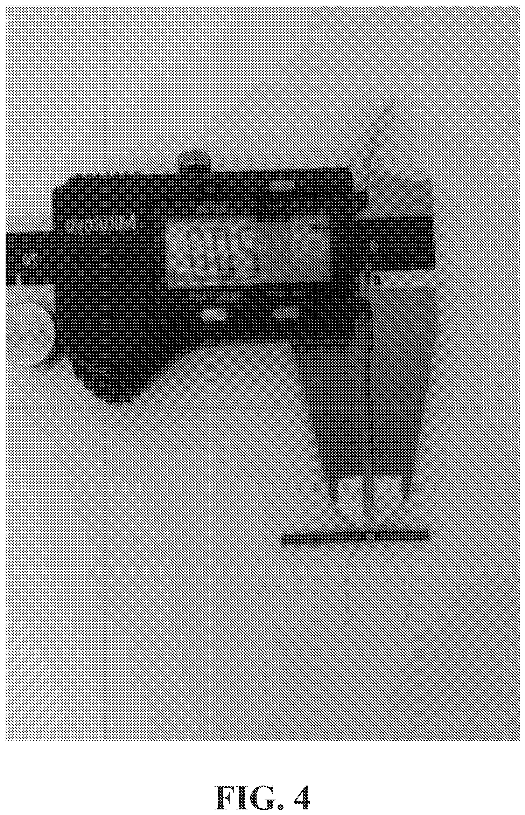

A typical active receiver loop coil has been designed and characterized for an example design using a ZnO film transducer. The coil shown in **Fig. 4** is made of 125µm wire and it impedance is measured as 3.5 + j79 Ω at 64MHz corresponding to 100nH inductance.

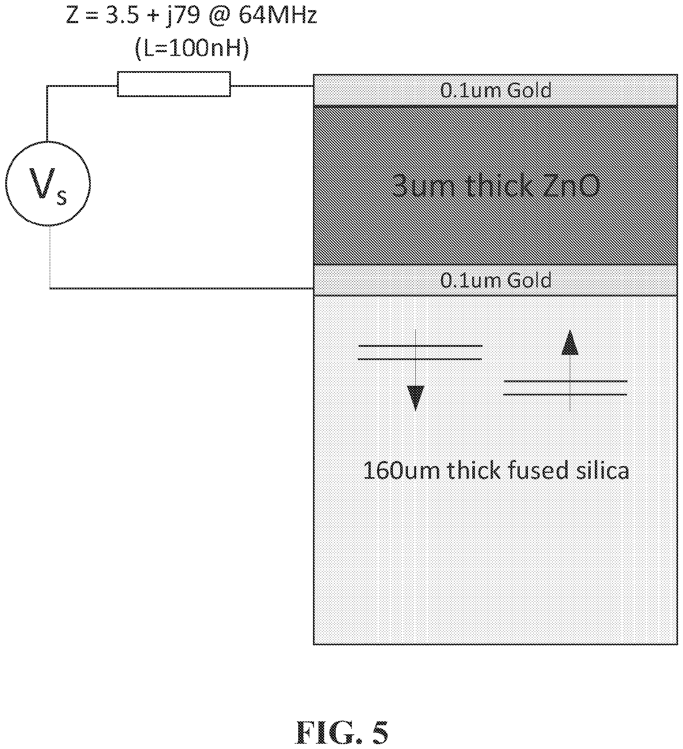

Based on this coil as the source impedance, a KLM model based acoustic simulation is performed using 2mm² area 3µm thick ZnO film on a 160µm diameter (core+ cladding) silica optical fiber for the system as shown in **Fig. 5****.**

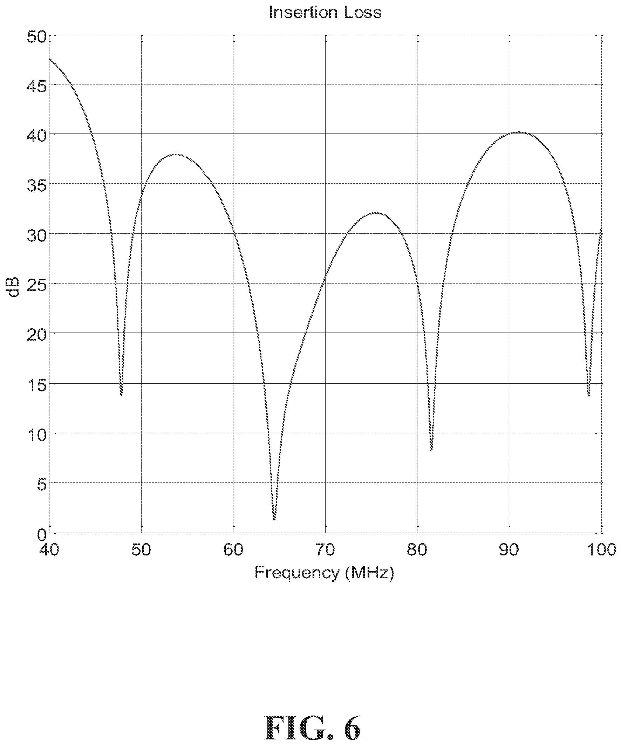

**Fig. 6** shows the insertion loss calculation which indicates several resonances due to finite thickness of the fused silica (longitudinal wave speed: 5960 m/s, density; 2200 kg/m³). An important point of the graph of **Fig. 6** is that the insertion loss can be close to 1.5dB around 64MHz, indicating that around this resonance 70% of the available power from the coil can be converted to ultrasound energy for acousto-optic modulation. Assuming that InW of electrical power is available from the coil and half of that power can be focused on a 4mm x 0.075mm area by the cylindrical focusing, a stress level of ∼700Pa will be generated on the FBG, which should result in 57dB SNR. This type of cylindrical focusing should be possible as the wavelength of longitudinal waves is about 90 µm at 64MHz.

In an exemplary embodiment, single mode optical fibers with desired diameter and phase shifted FBG will be designed and fabricated. The jacket of the fibers will be stripped at the distal end with the FBG sensor by chemical etching. A short section (4-5mm) of this region can be coated with 0.1-0.2 µm Ti-gold to from the bottom electrode of the ZnO transducer. The very distal end of the fiber can be coated by an optical absorber.

The fiber can then be placed in a ZnO sputtering station on a rotating stage that also allows for masking the electrode and ZnO deposition. After the top electrode deposition, the wire bonding will be used to make the electrical connections. The quality of the ZnO film, its density, coupling coefficient, will be evaluated before depositing films over the optical fibers. These parameters can be used to optimize the transducer design. For more detailed design of the acousto-optical coupler, a finite element method based analysis can be performed using appropriate materials and geometry.

For testing and use of the acousto-optic modulation of the present invention, and verification of the design parameters, the setup shown in **Fig. 7** can be used. In an exemplary embodiment, the signal transmitter/detector assembly **400** comprises a laser generated by, for example, a laser diode **410,** the laser coupled to the proximal end of the optical fiber that runs along an active catheter shaft, an optical coupler **420,** and a spectrum analyzer **430.**

The laser diode **410,** tunable in the 1520-1590nm range (for example TLB 6328, Velocity Tunable Diode Laser by New Focus) can be coupled to the proximal end of the optical fiber **320** through the optical coupler **420.** The optical power output spectrum can be monitored by the spectrum analyzer **430.** The laser will be tuned to a sharp slope of the reflected spectrum and the intensity of the reflected light from the FBG region will be monitored using a high speed photodetector **440** (for example Newport 1611FC-AC) to capture the transient MRI receive signals. System parameters such as strain sensitivity and SNR vs laser power can be characterized before integrating the optical fiber to a catheter prototype for further testing and fabrication.

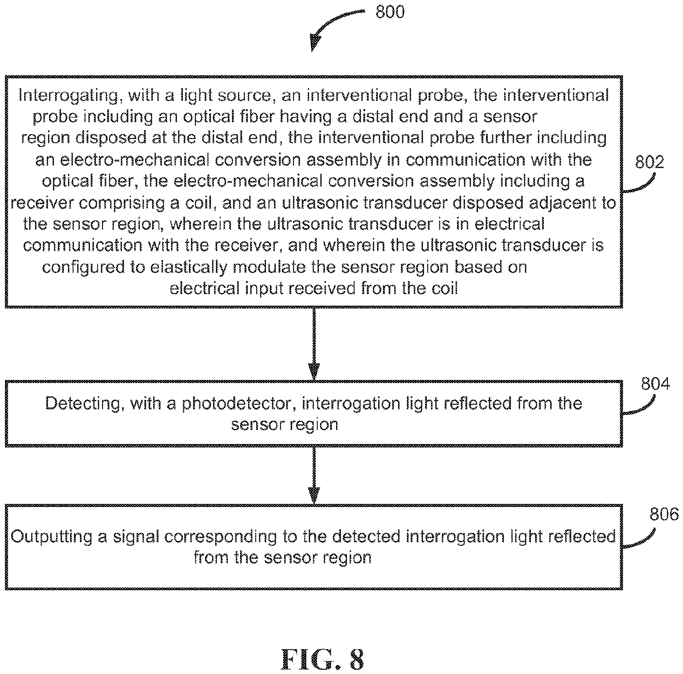

**Fig. 8** is a flow-diagram of a method **800,** in accordance with an example implementation of the disclosed technology. In block **802,** the method **800** includes interrogating, with a light source, an interventional probe, the interventional probe including an optical fiber having a distal end and a sensor region disposed at the distal end, the interventional probe further including an electro-mechanical conversion assembly in communication with the optical fiber, the electro-mechanical conversion assembly including a receiver comprising a coil, and an ultrasonic transducer disposed adjacent to the sensor region, wherein the ultrasonic transducer is in electrical communication with the receiver, and wherein the ultrasonic transducer is configured to elastically modulate the sensor region based on electrical input received from the coil. In block **804,** the method **800** includes detecting, with a photodetector, interrogation light reflected from the sensor region. In block **806,** the method **800** includes outputting a signal corresponding to the detected interrogation light reflected from the sensor region.

Numerous characteristics and advantages have been set forth in the foregoing description, together with details of structure and function. While the invention has been disclosed in several forms, it will be apparent to those skilled in the art that many modifications, additions, and deletions, especially in matters of shape, size, and arrangement of parts, can be made therein without departing from the scope of the invention and its equivalents as set forth in the following claims. Therefore, other modifications or embodiments as may be suggested by the teachings herein are particularly reserved as they fall within the breadth and scope of the claims here appended.

## Claims

1. A device comprising:
an optical fiber (320) including:
a distal end; and
a sensor region (312) disposed at the distal end; and
an electro-mechanical conversion assembly (200) in communication with the optical fiber (320), the electro-mechanical conversion assembly (200) including:
a receiver (210) comprising a coil (212); and
an ultrasonic transducer (222) disposed adjacent to the sensor region (312), wherein the ultrasonic transducer (222) is in electrical communication with the receiver (210), and wherein the ultrasonic transducer (222) is configured to elastically modulate the sensor region (312) based on electrical input received from the coil (212).

2. The device of claim 1, wherein the optical fiber (320) comprises at least one proximal end configured for coupling with an external light source for interrogation of the sensor region (312).

3. The device of claim 2, wherein the optical fiber (320) comprises at least one proximal end configured for coupling with a photodetector (440) to receive interrogation light reflected from the sensor region (312).

4. The device of claim 1, wherein at least the optical fiber (320) and the electro-mechanical conversion assembly (200) are configured to reduce radio frequency (RF)-induced heating of the device when utilized with Magnetic Resonance Imaging (MRI).

5. The device of claim 1, wherein the ultrasonic transducer (222) comprises at least two electrodes and a thin- film piezoelectric material (224) deposited on the optical fiber (320).

6. The device of claim 5, wherein the piezoelectric material comprises zinc oxide (ZnO).

7. The device of claim 1, wherein the sensor region (312) comprises a Fiber Bragg Grating (FBG) (314).

8. The device of claim 1, wherein a profile associated with the ultrasonic transducer (222) matches a profile associated with the optical fiber (320).

9. The device of claim 1, wherein the sensor region (312) comprises two or more Fiber Bragg Grating mirrors.

10. A system (100) comprising:
a device according to claim 1, the optical fiber (320) of the device further including at least one proximal end; and
a mechanical-optical conversion assembly (300) in communication with the at least one proximal end of the optical fiber (320), the mechanical-optical conversion assembly (300) including:
a light source coupled to the at least one proximal end of the optical fiber (320) and configured to interrogate the sensor region (312); and
a photodetector (440) coupled to the at least one proximal end of the optical fiber (320), the photodetector (440) configured to receive interrogation light reflected from the sensor region (312).

11. The system (100) of claim 10, wherein the system comprises an interventional probe configured for use with an MRI, and wherein at least the optical fiber (320) and the electro-mechanical conversion assembly (200) of the device are configured to reduce radio frequency-induced heating of the interventional probe.

12. The system (100) of claim 10, wherein the ultrasonic transducer (222) of the device comprises at least two electrodes and a thin- film piezoelectric material (224) deposited on the optical fiber (320).

13. A method (800) comprising:
Interrogating (802), with a light source, an interventional probe, the interventional probe comprising:
an optical fiber (320) having a distal end and a sensor region (312) disposed at the distal end; and
an electro-mechanical conversion assembly (200) in communication with the optical fiber (320), the electro-mechanical conversion assembly (200) including:
a receiver (210) comprising a coil (212), and
an ultrasonic transducer (222) disposed adjacent to the sensor region (312), wherein the ultrasonic transducer (222) is in electrical communication with the receiver (210), and wherein the ultrasonic transducer (222) is configured to elastically modulate the sensor region (312) based on electrical input received from the coil (212);
detecting (804), with a photodetector (440), interrogation light reflected from the sensor region (312); and
outputting (806) a signal corresponding to the detected interrogation light reflected from the sensor region (312).

14. The method (800) of claim 13, further comprising computing a temperature associated with the sensor region (312) based at least in part on a the detected interrogation light reflected from the sensor region (312).

15. The method (800) of claim 13, further comprising selectively adjusting a resonance associated with the coil (212), wherein selectively adjusting the resonance comprises optically switching a photoresistor or photodiode in communication with the coil (212).

## Patentansprüche

1. Vorrichtung, umfassend:
eine optische Faser (320), enthaltend:
ein distales Ende; und
einen Sensorbereich (312), der am distalen Ende angeordnet ist; und
eine elektromechanische Umwandlungsanordnung (200) in Kommunikation mit der optischen Faser (320), wobei die elektromechanische Umwandlungsanordnung (200) enthält:
einen Empfänger (210), der eine Spule (212) umfasst; und
einen Ultraschallwandler (222), der an den Sensorbereich (312) angrenzend angeordnet ist, wobei der Ultraschallwandler (222) in elektrischer Kommunikation mit dem Empfänger (210) ist und wobei der Ultraschallwandler (222) konfiguriert ist, den Sensorbereich (312) basierend auf dem elektrischen Eingang, der von der Spule (212) empfangen wird, elastisch zu modulieren.

2. Vorrichtung nach Anspruch 1, wobei die optische Faser (320) mindestens ein proximales Ende umfasst, das zur Kopplung mit einer externen Lichtquelle zum Abfragen des Sensorbereichs (312) konfiguriert ist.

3. Vorrichtung nach Anspruch 2, wobei die optische Faser (320) mindestens ein proximales Ende umfasst, das zur Kopplung mit einem Fotodetektor (440) konfiguriert ist, um Abfragelicht aufzunehmen, das vom Sensorbereich (312) reflektiert wird.

4. Vorrichtung nach Anspruch 1, wobei mindestens die optische Faser (320) und die elektromechanische Umwandlungsanordnung (200) konfiguriert sind, eine Radiofrequenz- (RF) induzierte Erwärmung der Vorrichtung zu verringern, wenn sie mit Magnetresonanztomographie (MRI) genutzt wird.

5. Vorrichtung nach Anspruch 1, wobei der Ultraschallwandler (222) mindestens zwei Elektroden und ein piezoelektrisches Dünnfilmmaterial (224), das auf der optischen Faser (320) angeordnet ist, umfasst.

6. Vorrichtung nach Anspruch 5, wobei das piezoelektrische Material Zinkoxid (ZnO) umfasst.

7. Vorrichtung nach Anspruch 1, wobei der Sensorbereich (312) ein Faser-Bragg-Gitter (FBG) (314) umfasst.

8. Vorrichtung nach Anspruch 1, wobei ein Profil, das dem Ultraschallwandler (222) zugeordnet ist, einem Profil entspricht, das der optischen Faser (320) zugeordnet ist.

9. Vorrichtung nach Anspruch 1, wobei der Sensorbereich (312) zwei oder mehr Faser-Bragg-Gitter-Spiegel umfasst.

10. System (100), umfassend:
eine Vorrichtung nach Anspruch 1, wobei die optische Faser (320) der Vorrichtung ferner mindestens ein proximales Ende enthält; und
eine mechanisch-optische Umwandlungsanordnung (300) in Kommunikation mit dem mindestens einen proximalen Ende der optischen Faser (320), die mechanisch-optische Umwandlungsanordnung (300) enthaltend:
eine Lichtquelle, die mit dem mindestens einen proximalen Ende der optischen Faser (320) gekoppelt ist und konfiguriert ist, den Sensorbereich (312) abzufragen; und
einen Fotodetektor (440), der mit dem mindestens einen proximalen Ende der optischen Faser (320) gekoppelt ist, wobei der Fotodetektor (440) konfiguriert ist, Abfragelicht aufzunehmen, das vom Sensorbereich (312) reflektiert wird.

11. System (100) nach Anspruch 10, wobei das System eine interventionelle Sonde umfasst, die zur Verwendung mit einem MRI konfiguriert ist, und wobei mindestens die optische Faser (320) und die elektromechanische Umwandlungsanordnung (200) der Vorrichtung konfiguriert sind, Radiofrequenz-induzierte Erwärmung der interventionellen Sonde zu verringern.

12. System (100) nach Anspruch 10, wobei der Ultraschallwandler (222) der Vorrichtung mindestens zwei Elektroden und ein piezoelektrisches Dünnfilmmaterial (224), das auf der optischen Faser (320) angeordnet ist, umfasst.

13. Verfahren (800), umfassend:
Abfragen (802), mit einer Lichtquelle, einer interventionellen Sonde, die interventionelle Sonde umfassend:
eine optische Faser (320) mit einem distalen Ende und einem Sensorbereich (312), der am distalen Ende angeordnet ist; und
eine elektromechanische Umwandlungsanordnung (200) in Kommunikation mit der optischen Faser (320), die elektromechanische Umwandlungsanordnung (200) enthaltend:
einen Empfänger (210), der eine Spule (212) umfasst, und
einen Ultraschallwandler (222), der an den Sensorbereich (312) angrenzend angeordnet ist, wobei der Ultraschallwandler (222) in elektrischer Kommunikation mit dem Empfänger (210) ist und wobei der Ultraschallwandler (222) konfiguriert ist, den Sensorbereich (312) basierend auf dem elektrischen Eingang, der von der Spule (212) empfangen wird, elastisch zu modulieren;
Erfassen (804), mit einem Fotodetektor (440), von Abfragelicht, das vom Sensorbereich (312) reflektiert wird; und
Ausgeben (806) eines Signals, entsprechend dem erfassten Abfragelicht, das vom Sensorbereich (312) reflektiert wird.

14. Verfahren (800) nach Anspruch 13, ferner umfassend Errechnen einer Temperatur, die dem Sensorbereich (312) zugeordnet ist, basierend mindestens teilweise auf dem erfassten Abfragelicht, das vom Sensorbereich (312) reflektiert wird.

15. Verfahren (800) nach Anspruch 13, ferner umfassend selektives Anpassen einer Resonanz, die der Spule (212) zugeordnet ist, wobei selektives Anpassen der Resonanz ein optisches Umschalten eines Fotowiderstands oder einer Fotodiode in Kommunikation mit der Spule (212) umfasst.

## Revendications

1. Dispositif comprenant:
une fibre optique (320) comprenant:
une extrémité distale; et
une région de capteur (312) disposée à l'extrémité distale; et
un ensemble de conversion électromécanique (200) en communication avec la fibre optique (320), l'ensemble de conversion électromécanique (200) comprenant:
un récepteur (210) comprenant une bobine (212); et
un transducteur à ultrasons (222) disposé de façon adjacente à la région de capteur (312), où le transducteur à ultrasons (222) est en communication électrique avec le récepteur (210), et où le transducteur à ultrasons (222) est conçu pour moduler élastiquement la région de capteur (312) en fonction d'une entrée électrique reçue depuis la bobine (212).

2. Dispositif selon la revendication 1, dans lequel la fibre optique (320) comprend au moins une extrémité proximale conçue pour s'accoupler avec une source de lumière extérieure pour l'interrogation de la région de capteur (312).

3. Dispositif selon la revendication 2, dans lequel la fibre optique (320) comprend au moins une extrémité proximale conçue pour s'accoupler avec un photodétecteur (440) afin de recevoir la lumière d'interrogation réfléchie depuis la région de capteur (312).

4. Dispositif selon la revendication 1, dans lequel au moins la fibre optique (320) et l'ensemble de conversion électromécanique (200) sont conçus pour réduire le chauffage induit par radiofréquence (RF) du dispositif en cas d'utilisation avec une imagerie par résonnance magnétique (IRM).

5. Dispositif selon la revendication 1, dans lequel le transducteur à ultrasons (222) comprend au moins deux électrodes et un matériau piézoélectrique à couche mince (224) déposé sur la fibre optique (320).

6. Dispositif selon la revendication 5, dans lequel le matériau piézoélectrique comprend de l'oxyde de zinc (ZnO).

7. Dispositif selon la revendication 1, dans lequel la région de capteur (312) comprend un Réseau de Bragg sur fibre (FBG) (314).

8. Dispositif selon la revendication 1, dans lequel un profil associé au transducteur à ultrasons (222) s'adapte à un profil associé à la fibre optique (320).

9. Dispositif selon la revendication 1, dans lequel la région de capteur (312) comprend deux miroirs ou plus de Réseau de Bragg sur fibre (FBG).

10. Système (100) comprenant:
un dispositif selon la revendication 1, la fibre optique (320) du dispositif comprenant en outre au moins une extrémité proximale; et
un ensemble de conversion mécanique-optique (300) en communication avec l'au moins une extrémité proximale de la fibre optique (320), l'ensemble de conversion mécanique-optique (300) comprenant:
une source de lumière couplée à l'au moins une extrémité proximale de la fibre optique (320) et conçue pour interroger la région de capteur (312); et
un photodétecteur (440) couplé à l'au moins une extrémité proximale de la fibre optique (320), le photodétecteur (440) étant conçu pour recevoir la lumière réfléchie depuis la région de capteur (312).

11. Système (100) selon la revendication 10, dans lequel le système comprend une sonde interventionnelle conçue pour être utilisée avec une IRM, et dans lequel au moins la fibre optique (320) et l'ensemble de conversion électromécanique (200) du dispositif sont conçus pour réduire le chauffage induit par radiofréquence de la sonde interventionnelle.

12. Système (100) selon la revendication 10, dans lequel le transducteur à ultrasons (222) du dispositif comprend au moins deux électrodes et un matériau piézoélectrique à couche mince (224) déposé sur la fibre optique (320).

13. Procédé (800) comprenant les étapes consistant à:
interroger (802), avec une source de lumière, une sonde interventionnelle, la sonde interventionnelle comprenant:
une fibre optique (320) ayant une extrémité distale et une région de capteur (312) disposée au niveau de l'extrémité distale; et
un ensemble de conversion électromécanique (200) en communication avec la fibre optique (320), l'ensemble de conversion électromécanique (200) comprenant:
un récepteur (210) comprenant une bobine (212); et
un transducteur à ultrasons (222) disposé de façon adjacente à la région de capteur (312), où le transducteur à ultrasons (222) est en communication électrique avec le récepteur (210), et où le transducteur à ultrasons (222) est conçu pour moduler élastiquement la région de capteur (312) en fonction d'une entrée électrique reçue depuis la bobine (212);
détecter (804), avec un photodétecteur (440), la lumière d'interrogation réfléchie depuis la région de capteur (312); et
fournir (806) un signal correspondant à la lumière d'interrogation détectée réfléchie depuis la région de capteur (312).

14. Procédé (800) selon la revendication 13, comprenant en outre l'étape consistant à calculer une température associée à la région de capteur (312) en fonction au moins en partie de la lumière d'interrogation détectée réfléchie depuis la la région de capteur (312).

15. Procédé (800) selon la revendication 13, comprenant en outre l'étape consistant à ajuster sélectivement une résonance associée à la bobine (212), où l'ajustement sélectif de la résonance comprend l'opération consistant à commuter optiquement une photorésistance ou une photodiode en communication avec la bobine (212).
